# EUROPÄISCHE PATENTSCHRIFT

(11) **EP 1 608 266 B1**
(45) Veröffentlichungstag und Bekanntmachung des Hinweises auf die Patenterteilung: **24.11.2010**
(21) Anmeldenummer: 04718958.4
(22) Anmeldetag: 10.03.2004
(51) Int. Cl.: A61B 5/15, A61B 10/00, A61M 1/02

(54) **VERFAHREN UND VORRICHTUNG ZUR GEWINNUNG VON NABELSCHNURBLUT**
METHOD AND DEVICE FOR RECOVERING BLOOD FROM AN UMBILICAL CORD
PROCEDES ET DISPOSITIFS POUR OBTENIR DU SANG OMBILICAL

(30) Priorität: 11.03.2003 DE 10310533
(43) Veröffentlichungstag der Anmeldung: 28.12.2005
(73) Patentinhaber: Fraunhofer-Gesellschaft zur Förderung der angewandten Forschung e.V., 80686 München (DE)
(72) Erfinder: ZIMMERMANN, Heiko, 66113 Saarbrücken (DE); BROMBACH, Till, 79618 Rheinfelden (DE); STIEGLITZ, Thomas, 66564 Ottweiler (DE); FUHR, Günter, 13187 Berlin (DE)
(74) Vertreter: Hertz, Oliver
(86) Internationale Anmeldenummer: PCT/EP2004/002461
(87) Internationale Veröffentlichungsnummer: WO 2004/080304

(56) Entgegenhaltungen:
- WO-A-92/03180
- WO-A-98/32475
- US-A1- 2001 025 170
- US-A1- 2002 002 355

## Beschreibung

Die vorliegende Erfindung betrifft ein Verfahren zur Gewinnung von Nabelschnurblut, bei dem Gewebe, welches das Nabelschnurblut enthält, in einer Kammer einem Überdruck ausgesetzt wird, unter dessen Wirkung das Nabelschnurblut in eine Auffangeinrichtung fließt, und eine Sammlereinrichtung zur Umsetzung eines derartigen Verfahrens.

Es ist bekannt, nach der Geburt eines Kindes und nach dessen Abnabelung von der Mutter aus der Nabelschnur und der Plazenta Blut (im folgenden: Nabelschnurblut) zu gewinnen. Nabelschnurblut enthält Stammzellen, die für therapeutische Anwendungen von Interesse sind (C. Regidor et al. in "Experimental Hematology" Bd. 27, 1999, S. 380-385). Nach der Sammlung erfolgt zunächst eine Lagerung in einer Blutbank (siehe E. Gluckman et al. in "Bone Marrow Transplantation" Bd. 22, Suppl. 1, 1998, S. S68-S74). Wesentlich für die spätere Verwendbarkeit einer Probe ist das Volumen des aufgefangenen Nabelschnurblutes. Bei jeder einzelnen Geburt wird angestrebt, ein möglichst großes Blutvolumen zu erhalten (siehe D. V. Surbek et al. in "Am. J. Obstet. Gynecol." Bd. 183, 2000, S. 218-221). Das gesammelte Probenvolumen muss in Relation zum Körpergewicht des Empfängers eine bestimmte Grenze überschreiten (siehe A. Wong et al. in "Bone Marrow Transplantation" Bd. 27, 2001, S. 133-138). Andernfalls muss die Probe wegen der geringen Chancen einer therapeutischen Verwertbarkeit verworfen werden. Wegen der bisher in der Regel kleinen Volumina ist die therapeutische Anwendung oft auf Kinder beschränkt. Ein typischer Wert für die in der Praxis angestrebte Mindestmenge beträgt 60 ml Nabelschnurblut.

Bisher ist es üblich, Nabelschnurblut in einem Blutbeutel aufzufangen, der nach der Abnabelung des Kindes über eine Punktionskanüle mit dem mütterlichen Ende der Nabelschnur verbunden wird. Die Punktionskanüle wird in die Nabelschnurvene (Vena umbilicalis) eingestochen, und das Blut fließt unter der Wirkung der Schwerkraft in den Blutbeutel. Dieses Verfahren hat mehrere Nachteile. Erstens steht nur ein relativ kurzes Zeitfenster zur Blutaufnahme zur Verfügung, die Sammlung des Blutes muss mit Beginn der Plazentation beendet werden. Das gewonnene Blutvolumen ist daher stark von den Bedingungen der Geburt und den fachlichen Fähigkeiten des Anwenders (zum Beispiel Arzt) abhängig.

Es sind verschiedene Techniken bekannt, ein möglichst großes Blutvolumen zu gewinnen (siehe U. Elchalal et al. in "Am. J. Obstet. Gynecol." Bd. 182, 2000, S. 227-232; S. J. Fasouliotis et al. in "European Journal of Obstetrics and Gynecology and Reproductive Biology" Bd. 90, 2000, S. 13-25; F. Bertolini et al. in "Journal of Hematotherapy" Bd. 4, 1997; S. 29-36). Beispielsweise ist vorgeschlagen worden, unter Druck in die Plazenta eine Spülflüssigkeit einzuleiten, um die Effektivität der Blutsammlung zu erhöhen, oder über die Punktionskanüle mit einer Spritze im Gewebe einen Unterdruck zu erzeugen. Mit diesen Techniken ist es jedoch nur möglich, die großen Gefäße des plazentaren Gewebes zu leeren. Die zahlreichen Kapillargefäße, welche die Plazenta durchsetzen, können nur beschränkt erreicht werden. Außerdem sind die beschriebenen Techniken umständlich und für routinemäßige Anwendungen ungeeignet.

Es ist auch bekannt, Nabelschnurblut erst nach der Plazentation zu gewinnen (siehe J. V. Linden et al. "Journal of Hematology" Bd. 6, 1997, S. 535-541; A. Wong et al. siehe oben). Dabei wird zwar der Zeitdruck bei der Blutsammlung vermieden. Dennoch sind die mit den herkömmlichen Techniken erzielbaren Blutvolumina beschränkt.

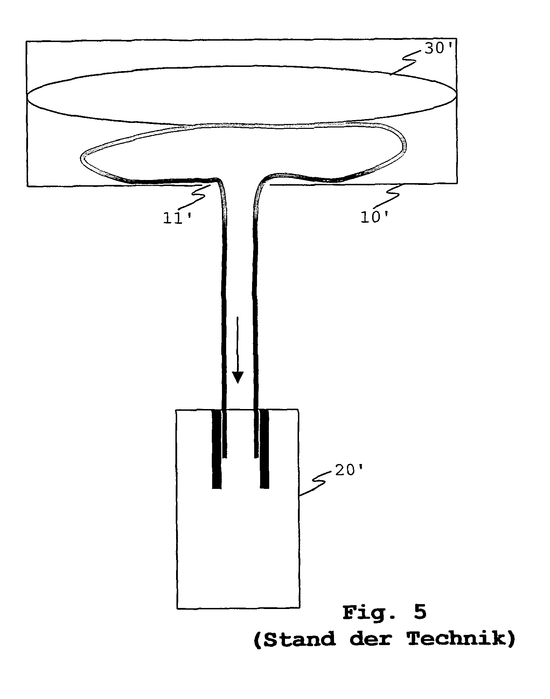

Es ist auch bekannt, die Plazenta einem mechanischen Druck auszusetzen, um mehr Blut zu gewinnen. Es wird beispielsweise in US 6,440,110 B2 vorgeschlagen, die Plazenta in einer Kammer 10' mit einer Öffnung 11' anzuordnen (siehe Figur 5), durch welche die Nabelschnur heraushängt. Um das Ausfließen des Blutes unter der Wirkung der Gravitation in einen Behälter 20' zu beschleunigen, wird die Plazenta mit einem aufblasbaren Ballon 30' oder einen entsprechenden hydraulisch betätigten Kolben zusammengequetscht. Dokumente WO-A-92/03180, US-A1-2001/025170 und WO-A-98/32475 beschreiben eine ähnliche Technik. Diese Technik besitzt eine Reihe von Nachteilen.

Das Hauptproblem besteht darin, dass das Gewebe durch das Zusammenquetschen geschädigt wird. In den gesammelten Blutproben sind unerwünschte Bestandteile enthalten, die auf geschädigtes Zellmaterial zurückzuführen sind. Des weiteren ist die Anwendung des in Figur 5 gezeigten Aufbaus erschwert, da die Nabelschnur ein heterogenes Gebilde ist, dessen Dicke von Fall zu Fall sehr verschieden sein kann. Dadurch können sich Probleme beim Einfädeln in die Öffnung 11' ergeben, und die Öffnung 11' erfordert ggf. eine Abdichtung, um ein Austreten von Gewebeteilen oder anderen Flüssigkeiten zu verhindern. Schließlich erfordert die Technik gemäss US 6,440,110 B2 zwingend die kombinierte Wirkung von Druck und Gravitation, um das Blut aufzufangen. Dadurch wird die Flexibilität des Aufbaus des Gerätes und die Anwendbarkeit bei verschiedenen Geburtsbedingungen beschränkt. Wegen der genannten Nachteile wird die beschriebene Technik mit einem mechanischen Stempeldruck in der Praxis nicht benutzt.

Die herkömmlichen Verfahren zur Gewinnung von Nabelschnurblut besitzen ferner die folgenden allgemeinen Nachteile. Bisher besteht keine Möglichkeit, Nabelschnurblut routinemäßig reproduzierbar unter standardisierten Bedingungen und unabhängig von Erfahrung des Arztes zu gewinnen. Außerdem sind die Bedingungen für ein steriles Arbeiten bisher nur schwer einhaltbar. Schließlich ist es besonders wichtig, dass durch die Entnahme des Nabelschnurbluts der Ablauf der Geburt nicht gestört und keinerlei Komplikationen verursacht werden.

Die Aufgabe der Erfindung ist es, ein verbessertes Verfahren zur Gewinnung von Nabelschnurblut bereitzustellen, mit denen die Nachteile der herkömmlichen Verfahren überwunden werden und die insbesondere die Gewinnung eines größeren Blutvolumens bei weitgehender Leerung der Plazenta, eine schonende Blutgewinnung und einen erweiterten Anwendungsbereich ermöglichen. Die Aufgabe der Erfindung ist es auch, verbesserte Vorrichtungen zur Gewinnung von Nabelschnurblut bereitzustellen, mit denen die Nachteile der herkömmlichen Techniken überwunden werden.

Diese Aufgaben werden durch Verfahren und Sammlervorrichtungen mit den Merkmalen gemäß den Patentansprüchen 1 oder 11 gelöst.

Vorteilhafte Ausführungsformen und Anwendungen der Erfindung ergeben sich aus den abhängigen Ansprüchen.

Das beanspruchte Verfahren basiert auf der allgemeinen technischen Lehre, das Gewebe, welches das Nabelschnurblut enthält, einem gleichförmigen Druck auszusetzen. Mit dem Druck wird über die Oberfläche des Gewebes ein im wesentlichen von allen Seiten wirkender, künstlicher Blutdruck erzeugt, durch den Blut auch aus den Kapillaren der Plazenta in die größeren Gefäße getrieben wird, ohne dass die Kapillaren oder Gewebeteile oder Zellen beschädigt werden. Das Gewebe ist mit mindestens einer Druckausgleicheinrichtung verbunden, durch die Blut in eine Umgebung verminderten Drucks, z. B. in eine Auffangeinrichtung abfließen kann. Mit der Erfindung wird ein Blutrest aus dem Kapillarsystem gewonnen, der bisher nicht oder nicht beschädigungsfrei zugänglich war und einen erheblichen Anteil des gewonnenen Blutvolumens bereitstellt.

Vorteilhafterweise können durch eine von der Umgebung, zum Beispiel von einer geschlossenen Kammer her allseitig und gleichförmig auf die Oberfläche des Gewebes gerichtete Druckwirkung die Schäden verhindert werden, die zum Beispiel bei der Technik gemäß Figur 5 auftreten können. Die Erfinder haben festgestellt, dass sich beim herkömmlich einseitigen Zusammenquetschen des Gewebes wie beim Zusammendrücken eines Schwammes im Gewebe ein Druckgradient aufbaut, der zu einer einseitigen Belastung im Gewebe führt, und dass die Gewebeteile nur beschränkt einseitige Belastungen im Gewebe tolerieren können. Im Gegensatz dazu können die Zellen allseitig wirkende Drucke überraschenderweise gut überstehen, ohne dass beispielsweise Gefäße kollabieren oder Gewebeteile beschädigt werden.

Der Druck wird vorzugsweise in der Kammer mit einem gasförmigen oder fließfähigen Medium als hydrostatischer Überdruck erzeugt. Der Druck wird auf die freie Oberfläche des Gewebes und von dieser auf das innere Kapillarsystem übertragen. Hierzu wird das Gewebe allseitig der Wirkung des Druckmediums ausgesetzt. Das gesamte Gewebe wird dem gleichen äußeren Druck ausgesetzt. Das Blut wird mit der Auffangeinrichtung gesammelt, in der ein geringerer Druck als in der Kammer herrscht. In der Auffangeinrichtung herrscht vorzugsweise Atmosphärendruck, entsprechend ist der Druck in der Kammer vorzugsweise höher als der Atmosphärendruck.

Als Druckausgleichseinrichtung kann eine Öffnung in einer Wand der Kammer vorgesehen sein, mit der mindestens ein Blutgefäß des Gewebes verbunden ist und an deren Außenseite außerhalb der Kammer sich die Auffangeinrichtung befindet. Des weiteren kann die Druckausgleichseinrichtung eine durch die Wand führende druckausgleichende Leitungsverbindung zwischen dem Gewebe in der Kammer und der Auffangeinrichtung außerhalb der Kammer aufweisen.

Wenn gemäß der ersten Variante das Nabelschnurblut vom Gewebe unmittelbar durch die mindestens eine Öffnung in der Wand der Kammer in die Auffangeinrichtung fließt, können sich Vorteile in Bezug auf eine einfache Bedienung und einen einfachen Aufbau der Sammlervorrichtung ergeben. Bevorzugt ist jedoch, wenn das Nabelschnurblut durch die mindestens eine Leitungsverbindung, die z. B. durch eine Punktionseinrichtung gebildet wird, von der Kammer in die Auffangeinrichtung fließt. Mit der Punktionseinrichtung kann vorteilhafterweise die druckausgleichende Verbindung mit der Auffangeinrichtung an einem bestimmten Ort im Gewebe, zum Beispiel dem Hauptgefäß in der Plazenta beginnen.

Wenn gemäß einer weiteren Ausführungsform der Erfindung der Überdruck in der Kammer mit einem unter Druck stehenden Gas erzeugt wird, können sich Vorteile durch verfügbare Druckquellen und für ein steriles Auffangen des Blutes ergeben. Wenn das Gas beispielsweise aus einer Druckflasche in die Kammer geleitet wird, kann vorteilhafterweise der Druck sehr genau eingestellt werden, so dass ein schonendes und reproduzierbares Sammeln des Blutes ermöglicht wird. Alternativ kann das Gas mit einer Pumpe unter Druck gestellt werden, was besonders für manuelle betätigte und/oder mobile Systeme von Vorteil ist.

Wenn gemäß einer weiteren Ausführungsform der Erfindung der Überdruck in der Kammer mit einer unter Druck stehenden Flüssigkeit erzeugt wird, kann dies vorteilhaft in Bezug auf die Behandlung des Gewebes mit Zusatzstoffen sein. Das fließfähige Medium (zum Beispiel eine physiologische Lösung, ein Antiseptikum oder ein Hydrogel) wird beispielsweise mit einer Pumpe in die Kammer geleitet. Ein besonders kompakter Aufbau ergibt sich, wenn die Pumpe durch einen in der Kammer bewegten Kolben gebildet wird.

Vorrichtungsbezogen basiert die Erfindung auf der allgemeinen technischen Lehre, eine Sammlereinrichtung zur Gewinnung von Nabelschnurblut dahingehend weiterzuentwickeln, dass sie mit einer Druckeinrichtung zur Erzeugung eines gleichförmigen, auf die Oberfläche des Gewebes mit Nabelschnurblut wirkenden Überdrucks ausgestattet ist oder verbunden werden kann.

Gemäß einer bevorzugten Ausführungsform der Erfindung umfasst die Sammlervorrichtung eine Kammer, in der das Gewebe positionierbar ist, welches das Nabelschnurblut enthält. Das Gewebe ist beispielsweise auf einem Kammerboden oder einer flexiblen Hängeinrichtung angeordnet. Die Kammer ist fest mit der Druckeinrichtung verbunden oder mit einem Druckanschluss ausgestattet, über den die Kammer zumindest während der Gewinnung des Nabelschnurblutes mit der Druckeinrichtung verbunden werden kann. Die Kammer ist des Weiteren mit der mindestens einen Druckausgleichseinrichtung ausgestattet, die insbesondere durch die Öffnung in der Kammerwand mit der druckausgleichenden Leitungsverbindung bereitgestellt wird.

Wenn gemäß einer Variante die Kammer starre Wände aufweist, können sich Vorteile in Bezug auf eine reproduzierbare Positionierung des Gewebes relativ zur Druckeinrichtung oder dem Druckanschluss einerseits und für die Punktion mit einer Punktionseinrichtung ergeben. Alternativ kann die Kammer durch einen Beutel mit einer flexiblen Wand gebildet werden, wobei sich Vorteile in Bezug auf mobile Anwendungen, zum Beispiel bei Hausgeburten ergeben.

Gemäß einer weiteren Variante kann die Kammer durch einen Beutel mit elastischen Wänden gebildet werden. Das Gewebe wird in die Kammer in einem (zum Beispiel durch einen äußeren Unterdruck) gedehnten Zustand aufgenommen. Nach Freigabe der Kammer entspannen sich deren Wände, wobei allseitig auf das Gewebe der gewünschte Überdruck ausgeübt wird. Das Blut fließt durch eine Öffnung oder eine Punktionseinrichtung ab.

Weitere Vorteile der Erfindung bestehen darin, dass die Erzeugung eines gleichförmigen Überdrucks ein relativ einfach steuerbares Prinzip darstellt, das auch unter klinischen Bedingungen ohne weiteres akzeptiert wird. Des weiteren kann ein erheblich höherer Druck eingestellt werden als bei herkömmlichen Techniken, so dass die Effektivität der Blutsammlung steigt. Durch die erfindungsgemäße Technik ist eine Volumensteigerung um bis zu 80 % möglich. Die Gewinnung eines größeren Blutvolumens pro Körpermasse der Spenderin hat zur Folge, dass grundsätzlich alle Spenden für eine weitere Anwendung insbesondere für therapeutische Zwecke nutzbar sind und auf ein Verwerfen von Spenden mangels Volumen verzichtet werden kann.

Die Entnahme des Nabelschnurblutes erfolgt nach dem Ende der Plazentation und stellt daher keinen Eingriff in den Geburtsablauf dar. Die Erfindung ist bei den verschiedensten Formen von Geburten anwendbar, zum Beispiel Wassergeburten. Das erfindungsgemäße Verfahren kann mit den herkömmlichen Sammlungstechniken kombiniert werden, indem beispielsweise zuerst nach der Abnabelung eine Punktion der Nabelschnur und nach der Entwicklung der Nachgeburt die erfindungsgemäße Sammlung erfolgen.

Die Anwendung der Erfindung ist nicht auf die Gewinnung von Nabelschnurblut beschränkt, sondern entsprechend auf die Gewinnung von Körperflüssigkeiten aus anderen menschlichen oder tierischen Organen oder Geweben anwendbar.

Weitere Vorteile und Einzelheiten bevorzugter Ausführungsbeispiele der Erfindung werden im Folgenden unter Bezug auf die beigefügten Zeichnungen beschrieben. Es zeigen:
- Figuren 1 bis 4:: schematische Schnittansichten verschiedener Ausführungsformen erfindungsgemäßer Sammler- vorrichtungen, und
- Figur 5:: eine Illustration einer herkömmlichen Vor- richtung zur Gewinnung von Nabelschnurblut.

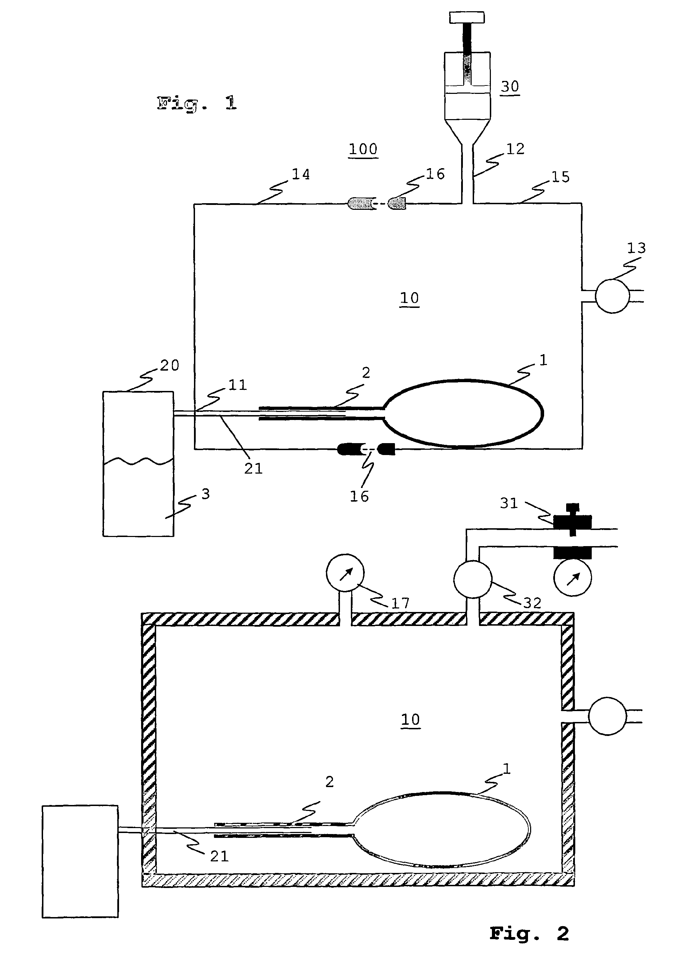

Die erfindungsgemäße Sammlervorrichtung 100 umfasst gemäß der in Figur 1 gezeigten Ausführungsform eine Kammer 10 zur Aufnahme der schematisch illustrierten Plazenta 1 mit der Nabelschnur 2, eine Auffangeinrichtung 20 zur Aufnahme von Nabelschnurblut 3 und eine Druckeinrichtung 30 zur Erzeugung eines hydrostatischen Überdruckes in der Kammer 10.

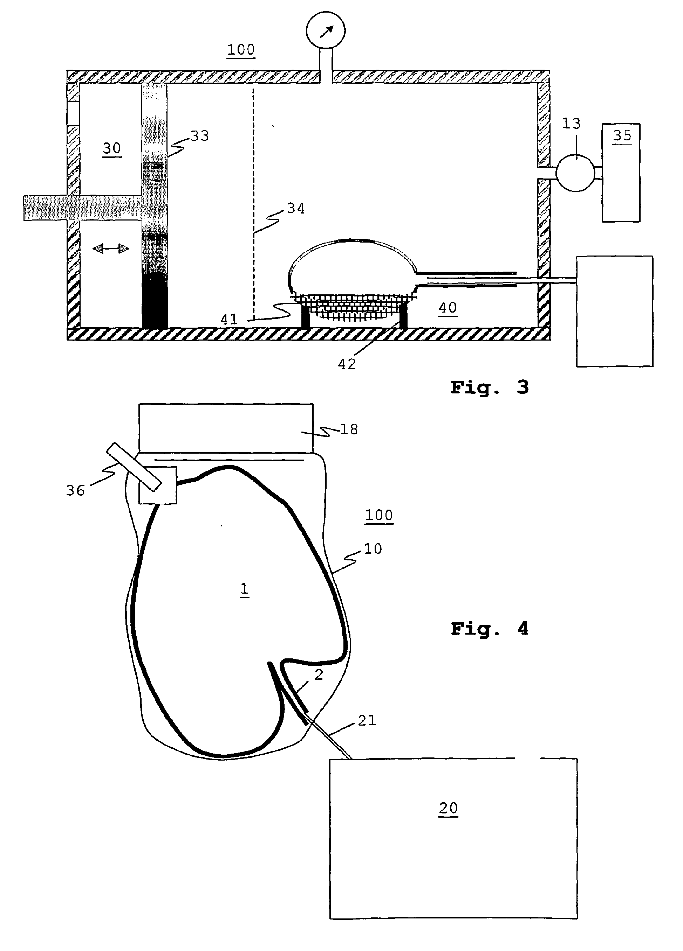

Die Kammer 10 ist ein geschlossenes, zum Beispiel kastenförmiges Behältnis, das eine Öffnung 11 zur Ableitung von Nabelschnurblut und insbesondere zur Durchführung einer Punktionseinrichtung 21, einen Anschluss 12 für die Druckeinrichtung 30 und ein Ablassventil 13 zum Druckausgleich aufweist. Die Kammer 10 ist mit zwei Kammerteilen 14, 15 gezeigt, die über ein umlaufendes Verbindungsstück 16 druckdicht miteinander verbunden werden können. Die Wände der Kammer bestehen bei dieser Gestaltung aus einem starren Material, das vorzugsweise durchsichtig ist, zum Beispiel einem transparenten Kunststoffmaterial, wie zum Beispiel PMMA, oder Glas. Alternativ ist die Kammer 10 ein flexibler Beutel (siehe Figur 4).

Als Auffangeinrichtung 20 kann jedes geeignete Behältnis zum Auffangen des Blutes verwendet werden, wie zum Beispiel ein Blutbeutel, eine Flasche oder eine Einrichtung für folgende Bearbeitungsschritte, wie zum Beispiel eine Zentrifugenkammer. Die Auffangeinrichtung 20 ist über die Punktionseinrichtung 21 mit dem Gewebe 1, 2 verbunden, die eine druckausgleichende Leitungsverbindung bildet. Sie stellt gleichzeitig den Druckausgleich während der Sammlung des Blutes dar. Hierzu besitzt die Auffangeinrichtung 20 einen Druckausgleich 22, wie zum Beispiel eine verschließbare Öffnung oder ein Ventil.

Die Auffangeinrichtung 20 ist vorzugsweise in vertikaler Richtung tiefer als das Gewebe 1, 2 in der Kammer 10 angeordnet, um bei der Sammlung des Blutes zusätzlich die Wirkung der Gravitationskraft auszunutzen. Es ist ein besonderer Vorteil der Erfindung, dass diese tiefere Anordnung jedoch nicht zwingend notwendig ist, da das Blut durch den Überdruck in der Kammer auch gegen die Gravitation bewegt werden kann.

Die Punktionseinrichtung 21 umfasst ein rohrförmiges Bauteil, insbesondere eine Punktionskanüle und einen Druckschlauch (Einzelheiten nicht dargestellt), das mit der Auffangeinrichtung 20 verbunden ist. Die Punktionseinrichtung 21 kann ortsfest im Boden oder in einer Wand der Kammer 10 angebracht sein. Die Punktion kann dann vorteilhafterweise automatisch beim Einlegen des Gewebes auf dem Kammerboden erfolgen.

Die schematisch illustrierte Druckeinrichtung 30 umfasst zum Beispiel eine Pumpe, wie zum Beispiel eine manuell betätigte Luftpumpe, oder ein ortsgebundenes oder mobiles Drucksystem (siehe unten).

Zur Durchführung des erfindungsgemäßen Verfahrens wird die Plazenta 1 mit der Nabelschnur 2, ggf. nach einer vorhergehenden herkömmlichen Punktions-Drainage, in den Kammerteil 15 gelegt und mit der Punktionseinrichtung 21 punktiert. Falls vorher die Punktions-Drainage durchgeführt wurde, wird die Plazenta mit der vorher benutzten Punktionseinrichtung verwendet, die in die Öffnung 11 eingesetzt wird. Die Positionierung des Gewebes 1, 2 erfolgt durch einfaches Ablegen auf dem Boden der Kammer 10. Anschließend wird das Verbindungsstück 16 geschlossen, der Druckschlauch der Punktionseinrichtung 21 mit der Auffangeinrichtung 20 verbunden und die Druckeinrichtung 30 betätigt.

Die Plazenta 1 ist ein nach der Plazentation flach geformtes Gewebe mit einer Dicke von rd. 2 bis 3 cm, einem Durchmesser von rd. 15 bis 20 cm und einem Gewicht von rd. 300 bis 600 g, wobei diese Werte individuell stark variieren können. Die Nabelschnur 2 besitzt eine von rd. Länge 30 bis 55 cm und einen Durchmesser von rd. 1 bis 2 cm. Die Kammergröße ist so gewählt, dass mindestens das Gewebevolumen aufgenommen werden kann, vorzugsweise jedoch größer im Bereich von zum Beispiel 1 bis 30 dm³.

Der Druck (Druckdifferenz gegenüber dem Atmosphärendruck) in der Kammer 10 wird beispielsweise auf einen Wert zwischen 0,05 bar bis 2 bar, vorzugsweise 0,05 bar bis 0,4 bar erhöht. Vorteilhafterweise wirkt der Druck vom Innenraum der Kammer 10 allseitig und gleichförmig auf die gesamte frei liegende Oberfläche des Gewebes 1, 2. Der Druck wird wegen der flachen Form des Gewebes auch auf die Unterseite in Kontakt mit dem Boden übertragen, so dass von diesem die entsprechende Gegenkraft aufgebaut wird. Im Unterschied zum herkömmlichen Aufbau gemäß Figur 5 ist der Druck wie ein Flüssigkeitsdruck allseitig ausgerichtet, es gibt keine Vorzugsrichtung, so dass auch die Zellen und Gefäße im Gewebe 1, 2 den hydrostatischen Druck als künstlichen Blutdruck erfahren. Unter der Wirkung des erhöhten Druckes werden alle Blutreste aus der Plazenta ausgetrieben und in die Auffangeinrichtung 20 überführt.

Nach Beendigung der Sammlung, was beispielsweise durch Beobachtung des Blutflusses festgestellt wird, kann die Auffangeinrichtung 20 von der Sammlervorrichtung 100 getrennt und der weiteren Anwendung zugeführt werden. Durch Betätigung des Ablassventils 13 wird der Überdruck im Innenraum der Kammer 10 abgebaut. Anschließend wird die Kammer 10 geöffnet und der Rest des Gewebes weiteren Untersuchungen zugeführt oder entsorgt.

Figur 2 zeigt eine alternative Ausführungsform der Erfindung mit einer starren Druckkammer 10, in die durch ein druckdicht verschließbares Fenster (nicht dargestellt) das Gewebe 1, 2 einlegbar ist. Die Verbindung mit der Auffangeinrichtung 20 erfolgt wiederum über die Punktionseinrichtung 21, die von außen in das Gewebe 1, 2 eingestochen wird. Als Druckeinrichtung ist eine externe Druckquelle (nicht dargestellt) vorgesehen, die beispielsweise durch ein stationäres Druckgassystem in einem Krankenhaus, eine Druckgasflasche, eine Pumpe oder einen Kompressor gebildet wird. Die Druckquelle ist über einen Druckminderer 31 und ein Absperrventil 32 mit der Kammer 10 verbunden. Mit dem Druckminderer 31, der vorzugsweise mit einem Manometer ausgestattet ist, kann vorteilhafterweise ein bestimmter Druckwert in der Kammer 10 eingestellt werden. Zur Kontrolle des tatsächlichen Druckes kann ein Manometer 17 vorgesehen sein, das in die Wand der Kammer 10 eingelassen ist. Die Verwendung des Aufbaus gemäß Figur 2 erfolgt wie oben unter Bezug auf Figur 1 beschrieben.

Bei der in Figur 3 gezeigten Ausführungsform der Erfindung ist die Druckeinrichtung 30 in die Kammer 10 integriert. Die Kammer besitzt einen vorzugsweise zylinderförmigen Innenraum, an dessen Innenmaß das Außenmaß eines Stempels 33 der Druckeinrichtung 30 angepasst ist. Um ein Auflaufen des Stempels 33 auf das Gewebe 1, 2 zu verhindern, ist ein Rückhalteelement 34, zum Beispiel in Form einer für das Druckmedium durchlässigen Wand, eines Netzes oder Gitters vorgesehen. Bei dem in Figur 3 gezeigten Aufbau wird vorzugsweise eine Flüssigkeit als Druckmedium verwendet. In diesem Fall kann das Ablassventil 13 mit einem Vorratsgefäß 35 zur Aufnahme des Druckmediums verbunden sein.

Figur 3 illustriert beispielhaft eine Hängeinrichtung 40, die zur Aufnahme des Gewebes 1, 2 vorgesehen sein kann. Die Hängeinrichtung 40 umfasst beispielsweise einen Träger in Form eines Netzes 41 oder einer flexiblen Folie mit Stützen 42, auf den das Gewebe mit Abstand vom Boden der Kammer 10 auflegbar ist und durch den die Druckübertragung auch von der Unterseite her besser ausgeglichen wird. Alternativ kann die Hängeinrichtung 40 einen frei in die Kammer reichenden Haken umfassen. Gemäß einer weiteren Abwandlung kann die Hängeinrichtung ohne die Stützen gebildet sein und lediglich eine Strukturierung (zum Beispiel Aufrauung, Netzstruktur, Stege/Nuten) auf dem Boden umfassen (Auflageeinrichtung).

Die Verwendung der in Figur 3 gezeigten Sammlervorrichtung erfolgt analog zu den oben beschriebenen Prinzipien. Der Stempel 33 wird nach Einlegen des Gewebes 1, 2 in die Kammer 10 manuell oder motorgetrieben betätigt, so dass sich das Innenvolumen der Kammer 10 verringert und der Druck entsprechend steigt.

Eine weitere Variante bei der Umsetzung der Erfindung ist eine mobile Ausführungsform der Sammlervorrichtung 100, die in Figur 4 illustriert ist. Es ist eine flexible Kammer 10 vorgesehen, die wie ein Blutbeutel aufgebaut ist und insbesondere ein Halteteil 18, zum Beispiel zum Aufhängen an einem Hilfsgerät, und eine druckdicht verschließbare Öffnung 19 aufweist. In das Folienmaterial der Kammer 10, das beispielsweise Polyethylen oder Polypropylen umfasst, ist als Druckeinrichtung ein Anschluss 36 für eine externe Druckquelle, wie zum Beispiel eine Luftpumpe, einen Kompressor oder eine Druckinfusion vorgesehen. Nach der Blutentnahme über die Punktionseinrichtung 21 in die Auffangeinrichtung 20 kann die Plazenta in der umhüllten Form gelagert und entsorgt werden. Vorteilhafterweise bildet die Kammer 10 bei dieser Ausführungsform ein billiges Einwegprodukt. Die in Figur 4 gezeigte Ausführungsform der Erfindung wird vorzugsweise bei Hausgeburten oder der Gewinnung von Nabelschnurblut oder Stammzellen aus tierischen Organismen verwendet.

Als Druckmedium kann allgemein ein Gas oder Gasgemisch (zum Beispiel Luft, inertes Gas, wie zum Beispiel Stickstoff) oder eine Flüssigkeit verwendet werden. Die Funktion der oben genannten Ausführungsformen erfolgt in beiden Fällen entsprechend. Als Druckquelle wird anstelle einer Druckgasquelle eine Druckflüssigkeitsquelle verwendet. Der Druckaufbau, die Druckmessung und der Druckabbau nach der Blutsammlung erfolgen in beiden Fällen gleich. Das Ablassventil 13 sollte allerdings bei der Verwendung von Flüssigkeiten als Druckmedium an einem unteren Teil der Kammer, vorzugsweise am Boden vorgesehen sein.

Bei der Umsetzung der Erfindung können die folgenden weiteren Abwandlungen vorgesehen sein. Es können mehrere Punktionseinrichtungen (Drainagen) und/oder weitere Öffnungen in der Kammer zum Abfließen von Blut vorgesehen sein. In der Kammer, der Punktionseinrichtung und/oder der Auffangeinrichtung können zusätzliche Messgeräte zur Erfassung von Parametern der gesammelten Flüssigkeit vorgesehen sein. In der Auffangeinrichtung kann das Blut mit zusätzlichen Substanzen, wie zum Beispiel Kryoadditiva, Antigerinnungsstoffen etc. versetzt werden, wie es an sich von herkömmlichen Punktionssystemen bekannt ist. Die Kammer kann eine Zylinder- oder Rohrform zum Beispiel mit kreisförmigem Querschnitt oder eine Kugel- oder Glockenform besitzen, um die Stabilität beim Druckaufbau zu erhöhen, wobei die Geometrie eines ggf. vorgesehenen Verbindungsstücks (siehe Figur 1) angepasst wird.

Die Erfindung ist nicht auf die vorstehend beschriebenen Ausführungsbeispiele beschränkt. Vielmehr ist eine Vielzahl von Varianten und Abwandlungen möglich, die von dem Erfindungsgedanken Gebrauch machen und deshalb ebenfalls in den Schutzbereich fallen.

## Patentansprüche

1. Verfahren zur Gewinnung von Nabelschnurblut, bei dem Gewebe (1, 2), welches das Nabelschnurblut enthält, in einer Kammer (10) einem Druck ausgesetzt wird, unter dessen Wirkung das Nabelschnurblut von dem Gewebe (1, 2) in der Kammer (10) in mindestens eine Auffangeinrichtung (20) fließt, **dadurch gekennzeichnet, dass**
- ein hydrostatischer Druck in der Kammer (10) allseitig gleichförmig auf das Gewebe (1, 2) wirkt,
- das Nabelschnurblut durch mindestens eine Druckausgleicheinrichtung in die Auffangeinrichtung fließt, und
- in der Auffangeinrichtung (20) ein geringerer Druck als in der Kammer (10) herrscht.

2. Verfahren nach Anspruch 1, bei dem das Nabelschnurblut durch mindestens eine Öffnung (11) in einer Wand der Kammer (10) in die Auffangeinrichtung (20) fließt.

3. Verfahren nach Anspruch 1, bei dem das Nabelschnurblut durch mindestens eine druckausgleichende Leitungsverbindung (21) (21) von dem Gewebe (1, 2) in der Kammer (10) in die Auffangeinrichtung (20) fließt.

4. Verfahren nach mindestens einem der vorhergehenden Ansprüche, bei dem der Druck in der Kammer (10) mit einem unter Druck stehenden Gas erzeugt wird.

5. Verfahren nach Anspruch 4, bei dem das Gas aus einer Druckflasche in die Kammer (10) geleitet wird.

6. Verfahren nach Anspruch 4, bei dem das Gas mit einer Pumpe (30) unter Druck gestellt wird.

7. Verfahren nach Anspruch 6, bei dem die Pumpe (30) manuell betätigt wird.

8. Verfahren nach mindestens einem der vorhergehenden Ansprüche 1 bis 3, bei dem der Druck in der Kammer (10) mit einer unter Druck stehenden Flüssigkeit erzeugt wird.

9. Verfahren nach Anspruch 8, bei dem die Flüssigkeit mit einer Pumpe in die Kammer (10) geleitet wird.

10. Verfahren nach Anspruch 9, bei dem die Pumpe durch einen in der Kammer (10) bewegten Kolben (33) gebildet wird.

11. Sammlervorrichtung (100) zur Gewinnung von Nabelschnurblut, mit:
- einer Kammer (10) zur Aufnahme von Gewebe, welches das Nabelschnurblut enthält,
- einer Druckeinrichtung (30) zur Erzeugung eines Druckes in der Kammer, und
- einer Auffangeinrichtung (20) zur Aufnahme des Nabelschnurbluts, das unter der Wirkung des Druckes aus der Kammer fließt, **dadurch gekennzeichnet, dass**
- die Druckeinrichtung (30) zur Erzeugung eines allseitig gleichförmig auf das Gewebe wirkenden hydrostatischen Druckes in der Kammer (10) eingerichtet ist, und
- bei Erzeugung des Drucks in der Auffangeinrichtung (20) ein geringerer Druck als in der Kammer (10) herrscht.

12. Sammlervorrichtung nach Anspruch 11, bei der eine Öffnung (11) in einer Wand der Kammer (10) vorgesehen ist, wobei durch die Öffnung (11) das Nabelschnurblut in die Auffangeinrichtung (20) fließt.

13. Sammlervorrichtung nach Anspruch 11, bei der eine druckausgleichende Leitungsverbindung (21) vorgesehen ist, durch die das Nabelschnurblut von der Kammer in die Auffangeinrichtung (20) fließt.

14. Sammlervorrichtung nach mindestens einem der vorhergehenden Ansprüche 11 bis 13, bei der die Druckeinrichtung (30) zur Erzeugung eines hydrostatischen Gasdrucks in der Kammer eingerichtet ist.

15. Sammlervorrichtung nach Anspruch 14, bei der die Druckeinrichtung eine Druckflasche umfasst.

16. Sammlervorrichtung nach Anspruch 14, bei der die Druckeinrichtung eine Pumpe (30) umfasst.

17. Sammlervorrichtung nach Anspruch 16, bei der die Pumpe manuell betätigbar ist.

18. Sammlervorrichtung nach mindestens einem der vorhergehenden Ansprüche 11 bis 13, bei der die Druckeinrichtung zur Erzeugung eines hydrostatischen Flüssigkeitsdrucks in der Kammer (10) eingerichtet ist.

19. Sammlervorrichtung nach Anspruch 18, bei der die Druckeinrichtung eine Pumpe umfasst, mit der eine Druckflüssigkeit in die Kammer (10) geleitet wird.

20. Sammlervorrichtung nach Anspruch 19, bei der die Pumpe durch einen in der Kammer (10) beweglichen Kolben (33) gebildet wird.

21. Sammlervorrichtung nach mindestens einem der vorhergehenden Ansprüche 11 bis 20, bei der die Kammer (10) eine starre Wand besitzt.

22. Sammlervorrichtung nach mindestens einem der vorhergehenden Ansprüche 11 bis 20, bei der die Kammer (10) durch einen Beutel mit einer flexiblen Wand gebildet wird.

## Claims

1. Method for recovering blood from an umbilical cord, in which tissue (1, 2) containing the blood from an umbilical cord is subjected to pressure in a chamber (10), under the effect of which pressure the blood from the umbilical cord flows from the tissue (1, 2) in the chamber (10) into at least one receiving means (20), **characterised in that**
- a hydrostatic pressure in the chamber (10) has a uniform effect on all sides of the tissue (1, 2),
- the blood from the umbilical cord flows into the receiving means via at least one pressure compensation device, and
- the pressure in the receiving means (20) is lower than that in the chamber (10).

2. Method according to claim 1, in which the blood from the umbilical cord flows into the receiving means (20) via at least one opening (11) in a wall of the chamber (10).

3. Method according to claim 1, in which the blood from the umbilical cord flows from the tissue (1, 2) in the chamber (10) into the receiving means (20) via at least one pressure-compensating conduit connection (21) (21).

4. Method according to at least one of the preceding claims, in which the pressure in the chamber (10) is generated using a pressurised gas.

5. Method according to claim 4, in which the gas is conveyed into the chamber (10) from a pressure vessel.

6. Method according to claim 4, in which the gas is pressurised using a pump (30).

7. Method according to claim 6, in which the pump (30) is operated manually.

8. Method according to at least one of the preceding claims 1 to 3, in which the pressure in the chamber (10) is generated using a pressurised fluid.

9. Method according to claim 8, in which the fluid is conveyed into the chamber (10) using a pump.

10. Method according to claim 9, in which the pump is formed by a plunger (33) which can be moved in the chamber (10).

11. Collecting device (100) for recovering blood from an umbilical cord, comprising:
- a chamber (10) for receiving tissue which contains the blood from an umbilical cord,
- a pressure means (30) for generating a pressure in the chamber, and
- a receiving means (20) for receiving the blood from the umbilical cord, which blood flows out of the chamber under the effect of the pressure, **characterised in that**
- the pressure means (30) is set up to generate a hydrostatic pressure, which has a uniform effect on all sides of the tissue, in the chamber (10), and
- when the pressure is generated, the pressure in the receiving means (20) is lower than that in the chamber (10).

12. Collecting device according to claim 11, in which an opening (11) is provided in a wall of the chamber (10), the blood from the umbilical cord flowing into the receiving means (20) via the opening (11).

13. Collecting device according to claim 11, in which a pressure-compensating conduit connection (21) is provided, via which the blood from the umbilical cord flows from the chamber into the receiving means (20).

14. Collecting device according to at least one of the preceding claims 11 to 13, in which the pressure means (30) is set up to generate a hydrostatic pressure in the chamber.

15. Collecting device according to claim 14, in which the pressure means comprises a pressure vessel.

16. Collecting device according to claim 14, in which the pressure means comprises a pump (30).

17. Collecting device according to claim 16, in which the pump is operated manually.

18. Collecting device according to at least one of the preceding claims 11 to 13, in which the pressure means is set up to generate a hydrostatic fluid pressure in the chamber (10).

19. Collecting device according to claim 18, in which the pressure means comprises a pump with which a pressurised fluid is conveyed into the chamber (10).

20. Collecting device according to claim 19, in which the pump is formed by a plunger (33) which can be moved in the chamber (10).

21. Collecting device according to at least one of the preceding claims 11 to 20, in which the chamber (10) has a rigid wall.

22. Collecting device according to at least one of the preceding claims 11 to 20, in which the chamber (10) is formed by a bag having a flexible wall.

## Revendications

1. Procédé pour l'obtention de sang ombilical, où un tissu (1, 2) contenant du sang ombilical est soumis dans une chambre (10) à une pression sous l'effet de laquelle le sang ombilical s'écoule du tissu (1, 2) dans la chambre (10) vers au moins un dispositif récepteur (20), **caractérisé**
- **en ce qu**'une pression hydrostatique dans la chambre (10) s'exerce de tous côtés uniformément sur le tissu (1, 2),
- le sang ombilical s'écoule dans le dispositif récepteur par l'intermédiaire d'au moins un dispositif d'équilibrage de pression,
et
- en ce qu'une pression inférieure à celle de la chambre (10) règne dans le dispositif récepteur (20).

2. Procédé selon la revendication l, où le sang ombilical s'écoule vers le dispositif récepteur (20) par au moins une ouverture (11) dans une paroi de la chambre (10).

3. Procédé selon la revendication 1, où le sang ombilical s'écoule du tissu (1, 2) dans la chambre (10) vers le dispositif récepteur (20) par au moins un raccord de conduite (21) équilibreur de pression.

4. Procédé selon au moins une des revendications précédentes, où la pression dans la chambre (10) est générée par un gaz sous pression.

5. Procédé selon la revendication 4, où le gaz est refoulé d'une bouteille sous pression dans la chambre (10).

6. Procédé selon la revendication 4, où le gaz est mis sous pression par une pompe (30).

7. Procédé selon la revendication 6, où la pompe (30) est actionnée manuellement.

8. Procédé selon au moins une des revendications 1 à 3, où la pression dans la chambre (10) est générée par un liquide sous pression.

9. Procédé selon la revendication 8, où le liquide est refoulé par une pompe dans la chambre (10).

10. Procédé selon la revendication 9, où la pompe est formée par un piston (33) mobile dans la chambre (10).

11. Système de collecte (100) pour l'obtention de sang ombilical, avec :
- une chambre (10) destinée à recevoir un tissu contenant du sang ombilical,
- un dispositif de pression (30) destiné à générer une pression dans la chambre, et
- un dispositif récepteur (20) destiné à recevoir le sang ombilical s'écoulant de la chambre sous l'effet de la pression, **caractérisé**
- **en ce que** le dispositif de pression (30) est réglé pour générer une pression hydrostatique qui s'exerce de tous côtés uniformément sur le tissu dans la chambre (10), et
- **en ce qu'**une pression inférieure à celle de la chambre (10) règne dans le dispositif récepteur (20) lorsque la pression est générée.

12. Système de collecte selon la revendication 11, où une ouverture (11) est prévue dans une paroi de la chambre (10), le sang ombilical s'écoulant par l'ouverture (11) dans le dispositif récepteur (20).

13. Système de collecte selon la revendication 11, où est prévu un raccord de conduite (21) équilibreur de pression, par lequel le sang ombilical s'écoule de la chambre vers le dispositif récepteur (20).

14. Système de collecte selon au moins une des revendications 11 à 13, où le dispositif de pression (30) est réglé pour générer une pression gazeuse hydrostatique dans la chambre.

15. Système de collecte selon la revendication 14, où le dispositif de pression comprend une bouteille sous pression.

16. Système de collecte selon la revendication 14, où le dispositif de pression comprend une pompe (30).

17. Système de collecte selon la revendication 16, où la pompe est actionnable manuellement.

18. Système de collecte selon au moins une des revendications 11 à 13, où le dispositif de pression est réglé pour générer une pression hydrostatique de liquide dans la chambre (10).

19. Système de collecte selon la revendication 18, où le dispositif de pression comprend une pompe au moyen de laquelle un liquide sous pression est refoulé dans la chambre (10).

20. Système de collecte selon la revendication 19, où la pompe est formée par un piston (33) mobile dans la chambre (10).

21. Système de collecte selon au moins une des revendications 11 à 20, où la chambre (10) est pourvue d'une paroi rigide.

22. Système de collecte selon au moins une des revendications 11 à 20, où la chambre (10) est formée par une poche avec une paroi flexible.
